# EUROPÄISCHE PATENTANMELDUNG

(11) **EP 3 020 705 A1**
(43) Veröffentlichungstag der Anmeldung: **18.05.2016**
(21) Anmeldenummer: 15194938.5
(22) Anmeldetag: 20.12.2011
(51) Int. Cl.: C07D 213/75, C07C 229/42, A61K 31/44, A61P 25/04, A61P 29/00, A61K 31/196

(54) **MULTIKOMPONENTENKRISTALLE AUS ([2-AMINO-6-(4-FLUORO-BENZYLAMINO)-PYRIDIN-3-YL]-CARBAMIDSÄUREETHYLESTER UND 2-[2-[(2,6-DICHLORPHENYL)-AMINO]-PHENYL]-ESSIGSÄURE**

(30) Priorität: 20.12.2010 DE 102010063612
(62) Teilanmeldung aus: 11805489.9
(71) Anmelder: TEVA GmbH, 89079 Ulm (DE)
(72) Erfinder: HOOCK, Christoph Martin, 01157 Dresden (DE); QADAN, Asal, 01097 Dresden (DE); TERHAAG, Bernd, 01326 Dresden (DE)
(74) Vertreter: Lang, Johannes

(57) **Zusammenfassung**

Die vorliegende Erfindung betrifft neuartige Multikomponentenkristalle, deren Herstellung sowie deren Verwendung zur Behandlung von Schmerzzuständen, insbesondere unklarer Genese, durch eine gleichzeitigen Wirkung auf Schmerzen, die durch Muskelverspannung oder degenerative Gelenkerkrankungen verursacht werden, als auch solche, die auf entzündlichen Prozessen beruhen. Die neuartigen Multikomponentenkristalle enthalten ([2-Amino-6-(4-fluoro-benzylamino)-pyridin-3-yl]-carbamidsäureethylester (Flupirtin) und 2-[2-[(2,6-Dichlorphenyl)-amino]-phenyl]-essigsäure (Diclofenac) als einzige Wirkstoffkombination und lassen sich durch Lösen der beiden Komponenten in einem molaren Verhältnis von 1,0 zu 0,9 bis 1,0 zu 1,1 in einem inerten organischen Lösungsmittel und anschließendes Auskristallisieren der Komplexverbindung herstellen.

## Beschreibung

Die vorliegende Erfindung betrifft neuartige Multikomponentenkristalle aus ([2-Amino-6-(4-fluoro-benzylamino)-pyridin-3-yl]-carbamidsäureethylester und 2-[2-[(2,6-Dichlorphenyl)-amino]-phenyl]-essigsäure, deren Herstellung sowie deren Verwendung in pharmazeutischen Zubereitungen. Die erfindungsgemäßen Multikomponentenkristalle eignen sich zur Behandlung von Schmerzzuständen, insbesondere unklarer Genese, durch eine gleichzeitige Wirkung auf Schmerzen, die durch Muskelverspannung oder degenerative Gelenkerkrankungen verursacht werden, als auch auf solche, die auf entzündlichen Prozessen beruhen.

([2-Amino-6-(4-fluoro-benzylamino)-pyridin-3-yl]-carbamidsäureethylester, auch als Flupirtin bekannt, ist ein zentral wirkendes Nichtopioid-Analgetikum, welches frei von den typischen Nebenwirkungen natürlicher oder synthetischer Opioide ist, wie beispielsweise Atemdepression, Verstopfung, Toleranzentwicklung, physische oder psychische Abhängigkeit oder Suchtgefahr. Flupirtin ist ein Wirkstoff, der einen erhöhten Muskeltonus normalisieren kann.

Flupirtin ist der Prototyp einer neuen Klasse von Analgetika mit neuen spezifischen und therapeutisch relevanten Eigenschaften. Der Wirkmechanismus von Flupirtin beruht dabei auf keinem direkten, sondern einem indirekten funktionellen NMDA-antagonistischen Effekt. Dieser Mechanismus resultiert in drei unterschiedlichen Wirkungsbildern: analgetisch, muskeltonusreduzierend und neuroprotektiv. Die verschiedenen Wirkungen von Flupirtin sind die Folge eines einzigen molekularen Wirkmechanismus, nämlich der Wirkung von Flupirtin als selektivem neuronalen Kaliumkanalöffner (selective neuronal potassium channel opener = SNEPCO) (siehe beispielsweise Kornhuber J et al. (1999); Kornhuber J et al. (1999a)), die ein neues Prinzip in der Schmerztherapie darstellt.

Als Folge dieser vielfältigen Wirkungen verfügt Flupirtin über ein einzigartiges und breites pharmakologisches Wirkspektrum. Flupirtin eignet sich zur Behandlung und Vorbeugung von akuten und chronischen Schmerzen, einschließlich neuropathischen Schmerzen, Nervenschmerzen, durch Krebserkrankungen verursachten Schmerzen, vasomotorischen und Migräne-Kopfschmerzen, Schmerzzuständen nach Operationen, nach Verletzungen, Verbrennungen, bei Dysmenorrhoe, Zahnschmerzen und arthritischen Schmerzen.

Antiinflammatorische Effekte des Flupirtins sind bei den üblichen analgetischen Dosierungen in nur geringem Ausmaß zu erwarten, da im Tierversuch erst bei hohen Dosen (> 30mg/kg Körpergewicht) eine signifikante antiinflammatorische Wirkung beobachtet wurde. Flupirtin ist vor allem wirksam bei der Behandlung und Vorbeugung von Schmerzen, die insbesondere durch Muskelverspannungen, Muskelspasmen und Muskelsteifheit bedingt sind. Es ist wirkt besonders gut bei der Behandlung von Rückenschmerzen, wobei die klare Differentialdiagnose zur Genese dieser spannungsbedingten Rückenschmerzen im Sinne von entzündlichen und/oder nicht-entzündlichen Ursachen im Einzelfall nicht immer eindeutig zu stellen ist

Flupirtinmaleat lässt sich mit verschiedenen Schmerzmitteln kombinieren, wie zum Beispiel mit Morphin (EP 0977736), mit Neurokinin-Antagonisten (WO 2007/128056), mit Tramadol (EP 1697005) oder auch Paracetamol (EP 207193)

2-[2-[(2,6-Dichlorphenyl)-amino]-phenyl]-essigsäure (Diclofenac) gehört zur Gruppe der nichtsteroidalen Antirheumatika (NSAIDS). Sie hemmen nichtselektiv die Cyclooxygenasen (COX) I und II, die im Organismus für die Bildung von entzündungsvermittelnden Prostaglandinen verantwortlich sind.

Diclofenac ist ein Arzneistoff, der als Natrium- oder Kaliumsalz bei leichten bis mittleren Schmerzen und Entzündungen eingesetzt wird, beispielsweise bei Rheuma, Prellungen, Zerrungen und Arthrose. Chemisch gehört es zu den Phenylessigsäuren. Diclofenac besitzt antipyretische, analgetische, antiphlogistische und antirheumatische Wirksamkeit.

Im Gegensatz zu Flupirtin sind von Diclofenac auch Kombinationspräparate erhältlich. Bekannte Markennamen sind beispielsweise Arthotec® (Diclofenac und Misoprostol), Combaren® (Codein und Diclofenac), Dolo-Neurobion® und Neurofenac® (Diclofenac und eine Vitamin B Kombination), Flectoparin® (Diclofenac und Heparin), Neodolpasse® (Diclofenac und Orphenadrin), (Diclofenac und Orphenadrin), Tobrafen (Diclofenac und Tobramycin), Voltamicin (Diclofenac und Gentamicin). Eine Arzneimittelkombination aus Diclofenac mit einem selektiven Kaliumkanalöffner (SNEPCO) ist jedoch noch nicht bekannt.

Die gleichzeitige Verabreichung von Flupirtinmaleat und verschiedenen NSAIDS ist in einer wissenschaftlichen Publikation erörtert (Diamantis, W; Gordon, R. Postgrad Med J 1987, 63 Suppl 3 (29-34). Schon in einer niedrigen Dosis (15 mg / kg, Mäuse, 35 mg / kg, Ratte) verstärkte Flupirtinmaleat in Kombination mit verschiedenen Dosierungen von NSAIDS Analgetika die antinociceptive (d. h. die Schmerzwahrnehmung hemmende) Aktivität von Paracetamol, Acetylsalicylsäure und Diclofenac.

Die synergistische Kombination von Flupirtinsalzen mit verschiedenen nicht-steroidalen Antiphlogistika ist in DE 3 665 538 beschrieben. Hier wurde gefunden, dass die Wirkung von Flupirtinmaleat überraschenderweise durch Kombination mit bestimmten nicht-steroidalen Antiphlogistika synergistisch gesteigert wird, wobei gleichzeitig die Wirkung der Antiphlogistika ebenfalls eine synergistische Steigerung erfährt. Hierbei wird Flupirtin als Maleat, Gluconat oder Hydrochlorid eingesetzt.

Die rein physikalische Mischung der Einzelkomponenten aus Flupirtinsalzen und Diclofenac-Natrium wie in DE 36 65 538.4 beschrieben zeigt unterschiedliche Löslichkeit und verschiedene Auflösungsgeschwindigkeit der Einzelkomponenten, so dass eine gleichmäßige Absorption aus dem Magen-Darm Trakt nicht erreicht werden kann. Des Weiteren kommt es durch die unterschiedlichen Kristallformen und verschieden Dichten der Wirkstoffe zu Entmischungen. Eine stabile Formulierung der Komponenten mit befriedigender Bioverfügbarkeit ist daher von diesen Mischungen nicht zu erwarten.

Durch die Bildung eines Multikomponentenkristalls aus einem Wirkstoff mit einem geeigneten zweiten Wirkstoff wird ein neuer Feststoff gebildet, dessen physikalische Eigenschaften sich von denen der Einzelkomponenten erheblich unterscheiden. Beeinflusste Parameter sind hier unter anderem Löslichkeit, Auflösungsgeschwindigkeit, Schmelzpunkt sowie Teilchenform und -größe.

EP 2 123 626 A1 beansprucht Cokristalle aus Duloxetin mit mindestens einem Cokristallbildner, der zur Schmerzbehandlung geeignet ist. Duloxetin ist ein Wirkstoff aus der Gruppe der selektiven Serotonin-Noradrenalin-Wiederaufnahmehemmer und wird u.a. in der Behandlung von Depressionen und Angststörungen eingesetzt. Als Cokristall ist ein Cokristall aus Duloxetin und S-Naprocen genannt.

Mulfikomponentenkristalle unterscheiden sich von der rein physikalischen Mischung der beiden Partner auch hinsichtlich der kristallographischen und spektroskopischen Eigenschaften. Geeignete Messmethoden zum Nachweis der Festkörpereigenschaften dieser neuen Verbindungen sind unter anderem Röntgenkristallstrukturanalyse am Pulver (XRPD), Festkörperkernresonanzspektroskopie (¹³C-CP/MAS-NMR) oder auch Differenzkalorimetrie (DSC).

Die Aufgabe der vorliegenden Erfindung ist es, eine neue Verbindung bereitzustellen, die sowohl über die einmalige anaigetische Wirkung des selektiven Kaliumkanalöffners (SNEPCO) Flupirtin als auch über die entzündungshemmende und analgetische Aktivität des Diclofenacs verfügt, und die sich leicht als pharmazeutische, feste Zubereitungsform formulieren lässt, ohne die typischen Probleme von physikalischen Mischungen wie unterschiedliche Bioverfügbarkeit oder Entmischung während des Produktionsprozesses zu zeigen.

Zudem soll die neue Arzneiform sowohl effektiv gegen Schmerzen, welche unter anderem durch Spannungen verursacht werden, als auch gegen solche, die durch entzündliche Prozesse verursacht werden, wirken, so dass ein behandelnder Arzt das Mittel auch bei Schmerzen unklarer Genese verabreichen kann und den Patienten dabei nicht zusätzlichen unakzeptablen Nebenwirkungen aussetzt.

Die Aufgabe wird erfindungsgemäß gelöst durch einen neuartigen Multikomponentenkristall aus ([2-Amino-6-(4-fluoro-benzylamino)-pyridin-3-yl]-carbamidsäureethylester (Flupirtin) und 2-[2-[(2,6-Dichlorphenyl)-amino]-phenyl]-essigsäure (Diclofenac), insbesondere durch einen Multikomponentenkristall, der die Komponenten ([2-Amino-6-(4-fluoro-benzylamino)-pyridin-3-yl]-carbamidsäureethylester (Flupirtin) und 2-[2-[(2,6-Dichlorphenyl)-amino]-phenyl]-essigsäure (Diclofenac) als einzige Wirkstoffkombination enthält.

Unter Multikomponentenkristall im Sinne der Erfindung wird ein Kristall verstanden, der entweder aus neutralen oder aus ionischen Komponenten besteht, wobei neutrale Komponenten für die Kristallisation eingesetzt werden, aber während der Kristallisation zum Multikomponentenkristall auch ionische Komponenten entstehen können. Ein Multikomponentenkristall ist also entweder ein Cokristall, ein Salz, ein Solvat oder eine Mischform, die sowohl Cokristall- als auch Salzanteile aufweist.

Ein Cokristall im Sinne der Erfindung ist eine kristalline Struktur, welche aus zwei oder mehr neutralen Verbindungen aufgebaut ist.

Im Unterschied zur rein physikalischen Mischung der Ausgangsstoffe zeichnet sich ein erfindungsgemäßer Multikomponentenkristall vorteilhaft durch veränderte physikalischchemische Eigenschaften aus, welche beispielsweise die Löslichkeit, Stabilität, Hygroskopizität, Handhabung und Tablettierbarkeit beeinflussen, und die seine eindeutige Charakterisierung ermöglichen.

Überraschend fanden die Erfinder, dass sich durch Erhitzen einer Flupirtin-Lösung mit einer Lösung von Diclofenac ein neuartiger Multikomponentenkristall, ein Cokristall aus ([2-Amino-6-(4-fluoro-benzylamino)-pyridin-3-yl]-carbamidsäureethylester (Flupirtin) und 2-[2-[(2,6-Dichlorphenyl)-amino]-phenyl]-essigsäure (Diclofenac), bilden lässt, der anschließend beim Abkühlen der Lösung auskristallisiert. Dies ist überraschend, da sich bisher unter den Carbonsäuren lediglich aus den unverzweigten Alkan-(bzw. Alken-)carbonsäuren Flupirtinsalze erhalten ließen. Selbst mit der strukturell ähnlichen Benzoesäure konnten zusammen mit Flupirtin keine Salze oder Co-Kristalle erhalten werden.

In einer Ausführungsform ist der erfindungsgemäße Multikomponentenkristall aus Flupirtin und Diclofenac gekennzeichnet durch ein Röntgenpulverdiffraktogramm, gemessen unter Verwendung von Cu-Kα₁-Strahlung und eines Johansson-Germanium-Einkristall-Monochromators mit einer Schrittweite von 0,00922° im Beugungswinkelbereich von 2θ = 3-80 °C, mit einem charakteristischen Peak bei 2θ = 6,1±0,2°.

Vorzugsweise ist der Multikomponentenkristall zusätzlich gekennzeichnet durch weitere charakteristische Peaks bei 2θ = 4,9±0,2°, 7,7±0,2°, und 19,6±0,2°. Besonders bevorzugt weist er zusätzlich weitere charakteristische Peaks bei 2θ = 11,1±0,2°, 12,3±0,2°, 14,6±0,2°, 20,9±0,2°, 22,5±0,2°, 24,2±0,2° und 24,8±0,2° auf.

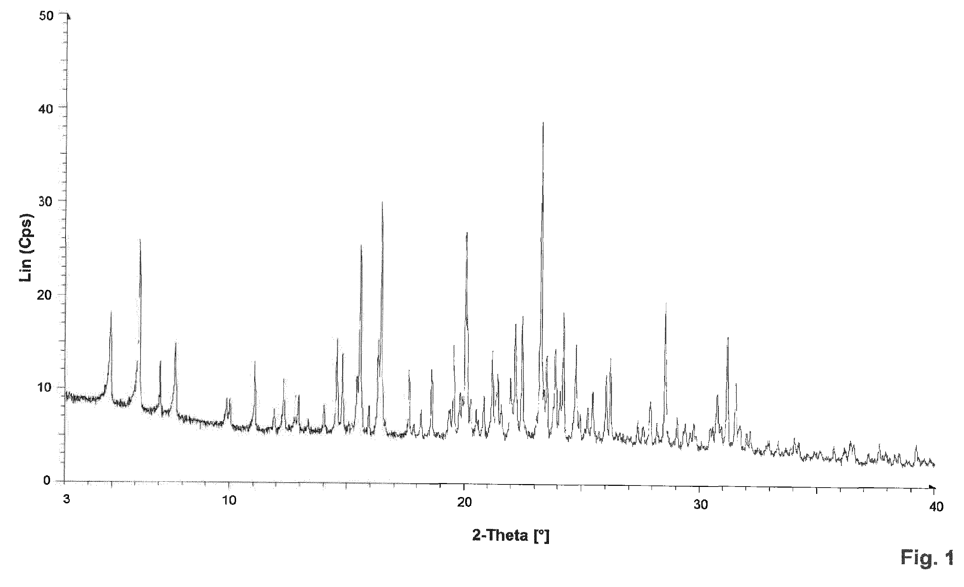

In einer bevorzugten Ausführungsform ist der erfindungsgemäße Multikomponentenkristall aus Flupirtin und Diclofenac durch ein Röntgenpulverdiffraktogramm, im Wesentlichen wie in Fig. 1 dargestellt, gekennzeichnet.

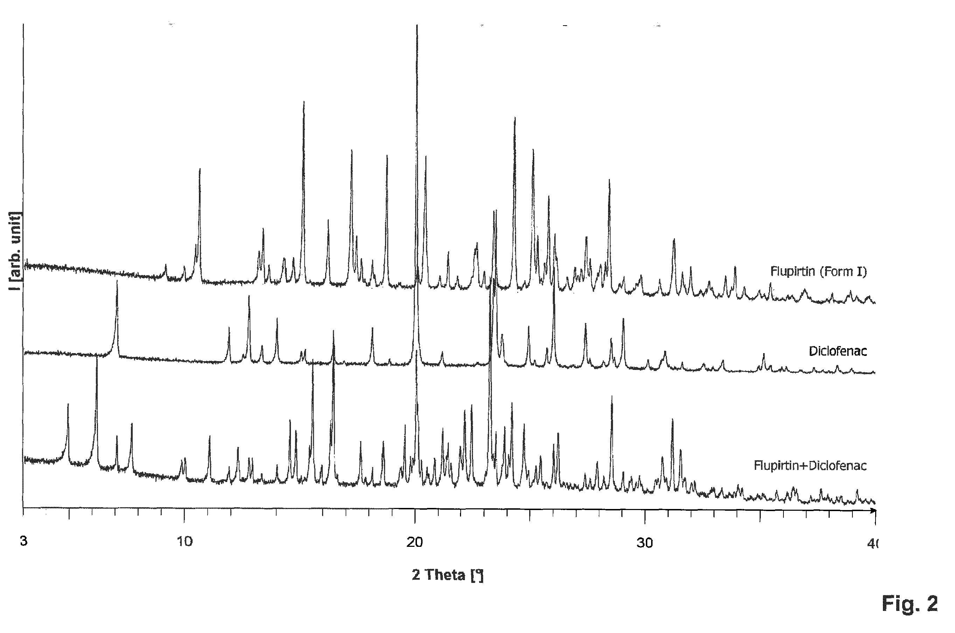

Desweiteren ist der erfindungsgemäße Multikomponentenkristall aus Flupirtin und Diclofenac vorzugsweise gekennzeichnet durch ein DSC(Differential scanning calorimetry)-Thermogramm, im Wesentlichen wie in Fig. 2 dargestellt. Er besitzt im Bereich von 50 bis 300 °C eine charakteristische Schmelzendotherme im Bereich von 100 bis 115 °C mit einer Onset-Temperatur von 96,9 ± 2 °C und mit einem Peakmaximum bei 107,2 °C ± 3 °C.

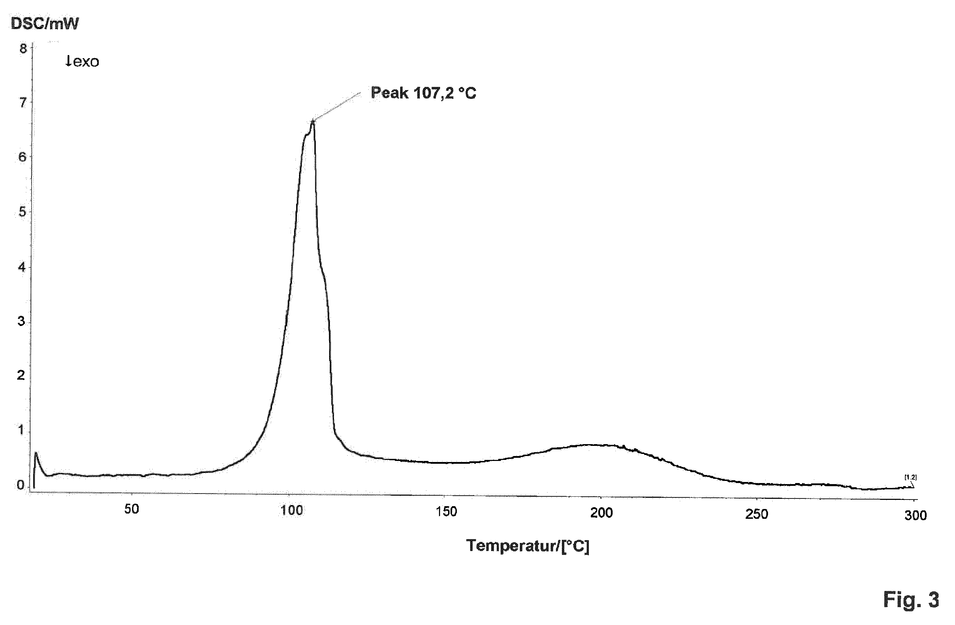

Vorzugsweise ist der erfindungsgemäße Multikomponentenkristall aus Flupirtin und Diclofenac darüber hinaus durch ein IR-Spektrum (KBr-Pressling) gekennzeichnet, welches charakteristische Peaks bei 3431±1 cm⁻¹, 3221±1 cm⁻¹, 2989±1 cm⁻¹, 1688±1 cm⁻¹, 1576±1 cm⁻¹, 1507±1 cm⁻¹, 1453±1 cm⁻¹, 1381±1 cm⁻¹, 1259±1 cm⁻¹, 1156±1 cm⁻¹, 1126±1 cm⁻¹, 1069±1 cm⁻¹, 832±1 cm⁻¹, und 776±1 cm⁻¹ aufweist. Vorzugsweise ist der erfindungsgemäße Multikomponentenkristall aus Flupirtin und Diclofenac gekennzeichnet durch ein IR-Spektrum, das im Wesentlichen mit Fig. 3 übereinstimmt.

Vorzugsweise ist der neuartige Multikomponentenkristall aus Flupirtin und Diclofenac aus äquimolaren Mengen der beiden Komponenten zusammengesetzt, wobei auch geringfügige Abweichungen von bis zu 10%, bevorzugt von bis zu 5 %, besonders bevorzugt von bis zu 2 %, des molaren Verhältnisses noch von der Erfindung umfasst sind, soweit sie makroskopisch eine einheitliche Verbindung ergeben. Das molare Verhältnis der Komponenten liegt also vorzugsweise im Bereich von 1:0,9 bis 1:1,1, bevorzugt von 1:0,95 bis 1:1,05, besonders bevorzug von 1:0,98 bis 1:1,02. Ganz besonders bevorzugt beträgt das molare Verhältnis der Komponenten 1:1.

Gegenstand der Erfindung ist auch ein Verfahren zur Herstellung des erfindungsgemäßen Multikomponentenkristalls aus Flupirtin und Diclofenac. Vorzugsweise wird dazu Flupirtin in der Basenform zusammen mit Diclofenac in ein geeignetes inertes organisches, bevorzugt aprotisches, Lösungsmittel, besonders bevorzugt Toluol, gegeben und unter Erwärmen, vorzugsweise auf eine Temperatur von 50 bis 80 °C, gelöst. Anschließend lässt man nach Abkühlen der Lösung auf Raumtemperatur den erfindungsgemäßen Multikomponentenkristall aus Flupirtin und Diclofenac als weißen, feinkristallinen Niederschlag auskristallisieren.

Gegenstand der Erfindung ist auch eine pharmazeutische Zubereitung, welche den erfindungsgemäßen Multikomponentenkristall enthält.

Die erfindungsgemäße pharmazeutische Zubereitung enthält neuartige Multikomponentenkristalle aus ([2-Amino-6-(4-fluoro-benzylamino)-pyridin-3-yl]-carbamidsäureethylester und 2-[2-[(2,6-Dichlorphenyl)-amino]-phenyl]-essigsäure gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Verfahren zur Bestimmung einer wirksamen Verabreichungsmenge der erfindungsgemäßen Zubereitung für therapeutische und prophylaktische Zwecke sind dem Fachmann geläufig. Um sowohl die analgetische Wirkung (u.a. durch Beeinflussung des Muskelspannungsschmerz) als auch die antiinflammatorische Wirkung der erfindungsgemäßen Zubereitung anwenden zu können, wird eine Tagesdosis von 50 mg bis 1000 mg, bevorzugt 200 mg bis 800 mg verabreicht.

Der neue erfindungsgemäße Multikomponentenkristall aus ([2-Amino-6-(4-fluoro-benzylamino)-pyridin-3-yl]-carbamidsäureethylester und 2-[2-[(2,6-Dichlorphenyl)-amino]-phenyl]-essigsäure hat aufgrund seiner physikalischen Eigenschaften überraschende Eigenschaften im Hinblick auf ihre galenische Verarbeitung zu festen Arzneiformen. Durch den neuen erfindungsgemäßen Multikomponentenkristall werden die in reinen physikalischen Mischungen üblicherweise vorhandenen arzneilich inaktiven Bestandteile der Basenkomponente (wie Maleat, Hydrochlorid, Mesilat, etc.) sowie der Säurekomponente (wie Natrium oder Kalium) vermieden, was bei fest oralen Darreichungsformen vorteilhaft zu einer Verringerung der zu verabreichenden Masse und des nötigen Volumens der Darreichungsform führt. Die einheitliche Form des Multikomponentenkristalls verhindert zudem vorteilhaft die Entmischung der Bestandteile während der Verarbeitung und erleichert so deren exakte Dosierung.

Eine Verabreichungseinheit kann beispielsweise 1- bis 5-mal, bevorzugt 1- bis 3-mal, vorzugsweise 2-mal täglich verabreicht werden.

Die erfindungsgemäße pharmazeutische Zubereitung kann auf verschiedenen, aus Pharmazie oder Medizin bekannten Wegen verabreicht werden, beispielsweise oral, parenteral, intraperitoneal, intravenös, sublingual, intramuskulär, rektal, transdermal, subkutan, intraadiposal, intraartikulär oder intrathekal. Bevorzugt ist die orale Verabreichung der pharmazeutischen Zubereitung.

Für die orale Verabreichung geeignete feste Verabreichungsformen umfassen Tabletten, Brausetabletten, Kapseln, Weichkapseln, Pillen, Pulver, Granulate, Pellets und dergleichen.

Die Freisetzung der Wirkstoffe aus der erfindungsgemäßen pharmazeutischen Zubereitung kann schnell oder verzögert erfolgen.

Als pharmazeutisch annehmbarer Carrier bzw. Füllstoff sind verschiedene gebräuchliche Hilfsstoffe wie zum Beispiel Zellulosederivate, Stärkederivate, Laktose, Mannitol, Dextrose, Saccharose, Calciumcarbonat, Magnesiumoxid, Magnesiumstearat, Talk, Stärke, Gelatine, Gummi arabicum oder dergleichen, oder gebräuchliche inerte Lösungsmittel einsetzbar.

Feste Verabreichungsformen umfassen gegebenenfalls weitere gebräuchliche Hilfsstoffe wie Fließmittel, Bindemittel, Füllmittel (wie z. B. Siliziumdioxid, insbesondere poröses amorphes Siliciumdioxid "Syloid", Carbomer, Guaran, Cellulose, mikrokristalline Cellulose, die Celluloseether, Celluloseester, Polyvinylpyrrolidon sowie Copovidone), Trennmittel, Gleitmittel, Sprengmittel (wie z. B. quervernetztes Polyvinylpyrrolidon, Natriumcarboxymethylstärke, Natriumcarboxy-methylcellulose, Stärke, Croscarmellose-Natrium, Crospovidon, Guaran, Primogel), Antioxidantien, Geschmacksstoffe, Farbstoffe, Lösungsvermittler (wie z. B. Cyclodextrine und Cyclodextrinderivate) und/oder Emulgatoren (wie z. B. Lecithin, Pektin).

Die festen Verabreichungsformen können mit Sucrose, mit einem Cellulosederivat, Polyacrylatderivat, Phthalatderivat oder anderen geeigneten Substanzen überzogen sein, oder sie können so behandelt sein, dass sie eine verlängerte oder verzögerte Wirkung haben und so, dass sie eine vorbestimmte Menge eines aktiven Wirkstoffs kontinuiertich freisetzen.

Die Erfindung umfasst daher auch eine pharmazeutische Zubereitung, die gekennzeichnet ist durch einen Polymerfilmüberzug, der als Retardkomponente wirkt und gegebenenfalls Trennmittel, Bindemittel, Pigmente oder andere pharmazeutische Hilfsstoffe enthält. Vorzugsweise enthält der Polymerfilmüberzug mindestens ein Polymer ausgewählt aus Methacrylsäure, Methacrylester (wie Eudragit®L und/oder Eudragit®S), Copolymerisate aus Acryl- und Methacrylsäureestern, Copolymerisate aus Acrylsäure und Methacrylsäure sowie auch deren Estern, oder Mischungen davon.

Durch Auflösung oder Suspendieren können auch flüssige Formulierungen erhalten werden, die zur Infusion oder Injektion geeignet sind.

Als nichtwässrige Lösungsmittel sind beispielsweise Propylenglycol, Polyethylenglycol und/oder organische Ester wie Ethyloleat einsetzbar. Beispiele für Öle sind Petroleum sowie tierische, pflanzliche oder synthetische Öle wie z. B. Erdnussöl. Rizinusöl, Sesamöl, Baumwollsaatöl, Maisöl, Weizenkeimöl, Sojaöl, Mineralöl. Olivenöl, Sonnenblumenöl oder Lebertran, insbesondere Kabeljau-Leberöl.

Durch Vermischen mit nicht wässrigen Lösungsmitteln oder Ölen lassen sich viskose bis halbfeste Formulierungen erzielen, die besonders gut in Weichkapseln abgefüllt werden können. Diese Kapseln sind trotz ihres relativ hohen Wirkstoffgehaltes von 100 bis 800mg aufgrund ihrer elastischen Hülle und Konsistenz des Inhaltes leicht zu schlucken.

Für den erfindungsgemäßen neuen Multikomponentenkristall aus den Wirkstoffen Flupirtin und Diclofenac erwies sich eine Mischung Glykolderivaten wie z. B. Diethylenglycolmonoethylether oder Polyethylenglykol, Tensiden wie z.B. Glycerinderivate sowie Verdickern wie z. B. Polyvinylpyrrolidon als geeigneter Trägerstoff für eine Weichkapselformulierung. Neutrale Lösungsvermittler (Detergenzien und Tenside) wie Glyceride (auch Polyoxylglyceride), Polyalkylenglykolether Caprylocaproyl oder Zuckerester stabilisieren und homogenisieren die Mischungen mit den erfindungsgemäßen Wirkstoffen.

Durch die geringe Polarität des erfindungsgemäßen neuen Multikomponentenkristalls aus den Wirkstoffen Flupirtin und Diclofenac eignen sich flüssige und halbfeste Formulierungen auch für die transdermale Applikation, ganz im Gegensatz zu einer Mischung der Komponenten Flupirtinmaleat und Diclofenac-Natrium. Besonders geeignet als Träger für den erfindungsgemäßen Multikomponentenkristall aus den Wirkstoffen Flupirtin und Diclofenac ist eine Mischung umfassend ein Carbomer, ein oder mehrere Fettsäureester, einem Polyethylenglykol und einen oder mehrere niedermolekulare Alkohole.

Überraschenderweise kann nach dem in DE 19 705 555 A1 offenbarten Verfahren durch Vermischen des Wirkstoffes mit porösem amorphen Siliciumdioxid ein Granulat erhalten werden, das sich problemlos zu Tabletten mit bis zu 600 mg Wirkstoff verpressen lässt. In einer Ausführungsform der Erfindung ist daher der erfindungsgemäße Multikomponentenkristall aus den Wirkstoffen Flupirtin und Diclofenac in einer Tablette enthalten, die vorzugsweise auch einen pharmazeutisch annehmbaren Filler enthält, wie beispielsweise amorphes Siliciumdioxid. Bevorzugt sind in einer Tablette 0,5 - 8 Gew.-% poröses amorphes Siliciumdioxid enthalten, bezogen auf das Gesamtgewicht der Tablette.

Die synergistische Wirkung der beiden Komponenten Flupirtin und Diclofenac lässt sich mit den in DE 36 65 538 verwendeten Methoden am Tiermodell zeigen. Die dort offenbarten Methoden werden hiermit als Referenz einbezogen.

Die mit den erfindungsgemäßen Cokristallen (Salzen) erzielten Wirkungen im Schmerz- und Entzündungsmodell sind qualitativ den Test-Werten von DE 36 65 538 gleichzusetzen. In Verbindung mit den Vorteilen der Formulierung ist der erfindungsgemäße Multikomponentenkristall aus den Wirkstoffen Flupirtin und Diclofenac der rein physikalischen Mischung der Einzelkomponenten deutlich überlegen.

Bestandteil der Erfindung ist auch die Verwendung des erfindungsgemäßen Multikomponentenkristalls zur Behandlung und Vorbeugung von akuten und chronischen Schmerzen, einschließlich neuropathischer Schmerzen, Rückenschmerzen, Nervenschmerzen, durch Krebserkrankungen verursachte Schmerzen, vasomotorische und/oder Migräne- oder spannungsbedingte Kopfschmerzen, Schmerzzuständen nach Operationen, nach Verletzungen, Verbrennungen, Verätzungen, bei Dysmenorrhoe, Vorbeugung von Muskelverspannung, Zahnschmerzen und arthritischen Schmerzen soweit eine inflammatorische Komponente des Schmerzes nicht ausgeschlossen ist. Die bevorzugte Anwendungsdauer reicht von 1-2 Tagen bis zu 6 Wochen.

Durch die vorteilhafte kombinierte Wirkung gegen nicht entzündungsbedingten Schmerz (wie z. B. muskelspannungsbedingten Schmerz) und Entzündungsschmerz ist der neue Wirkstoff besonders gut für Behandlung von Schmerzen des Bewegungsapparates unklarer Genese, einschließlich des Rückens einsetzbar, da hier bekanntermaßen besonders häufig entzündliche Prozesse und Muskelverspannungen auftreten.

Es können beispielsweise Tabletten verschiedener Größe hergestellt werden, beispielsweise mit einem Gesamtgewicht von etwa 50 - 800 mg. Sie enthalten den komplexen Wirkstoff in den vorgenannten Mengen und übliche Trägerstoffe und/oder Verdünnungsmittel und/oder Hilfsstoffe. Diese Tabletten können auch zur Verabreichung von Teildosen vorgesehen sein. In entsprechender Weise können beispielsweise auch andere Zubereitungen, wie zum Beispiel Gelatinekapseln oder Retardformen formuliert werden.

Für eine schnelle Auflösung und bessere Bio-Verfügbarkeit kann der neue erfindungsgemäße Multikomponentenkristall aus den Wirkstoffen Flupirtin und Diclofenac auch in Weichkapseln verabreicht werden. Die Wirkstoffe sind hier homogen in einer Softgel Matrix verteilt, das heißt suspendiert, gelöst oder teilweise gelöst. Trotz zusätzlicher Inhaltsstoffe sind diese Kapseln aufgrund ihrer weichen Hülle gut zu schlucken.

Anhand nachfolgender Figuren und Ausführungsbeispiele wird die Erfindung näher erläutert, ohne diese einzuschränken.

Dabei zeigen
Fig. 1 ein Röntgenpulverdiffraktogramm des Multikomponentenkristalls aus Flupirtin und Diclofenac,
Fig. 2 die Röntgenpulverdiffraktogramme von Diclofenac, von Flupirtin und des Multikomponentenkristalls aus Flupirtin und Diclofenac im Vergleich,
Fig. 3 ein DSC(Differential Scanning Calorimetry)-Thermogramm des Multikomponentenkristalls aus Flupirtin und Diclofenac,
Fig. 4 ein IR-Spektrum des erfindungsgemäßen Multikomponentenkristalls aus den Wirkstoffen Flupirtin und Diclofenac,
Fig. 5 ein 1H-NMR-Spektrum des erfindungsgemäßen Multikomponentenkristalls aus den Wirkstoffen Flupirtin und Diclofenac und
Fig. 6 ein ¹³C-CPMAS-NMR-Spektrum des erfindungsgemäßen Multikomponentenkristalls aus den Wirkstoffen Flupirtin und Diclofenac.

### Ausführungsbeispiele

### Beispiel 1: Herstellung eines erfindungsgemäßen Multikomponentenkristalls aus ([2-Amino-6-(4-fluoro-benzylamino)-pyridin-3-yl]-carbamidsäureethylester und 2-[2-[(2,6-Dichlorphenyl)-amino]-phenyl]-essigsäure

Unter Argonatmosphäre wurden 12,0 g ([2-Amino-6-(4-fluoro-benzylamino)-pyridin-3-yl]-carbamidsäureethylester (Flupirtin) und 11,8 g 2-[2-[(2,6-Dichlorphenyl)-amino]-phenyl]-essigsäure (Diclofenac) zu 500 ml Toluol gegeben und die Mischung auf 60°C erwärmt und für weitere 30 min bei 60 °C gehalten. Nach Abkühlen auf 20 °C wird die Lösung über Nacht stehen gelassen. Dabei bildete sich ein weißer, feinkristalliner Niederschlag aus. Dieser wurde abfiltriert und im Vakuum 12 h getrocknet.
Als Produkt werden 15,7 g (66 %) weiße Kristallnadeln erhalten. Das Produkt schmilzt bei einer Temperatur von 101 bis 103°C.

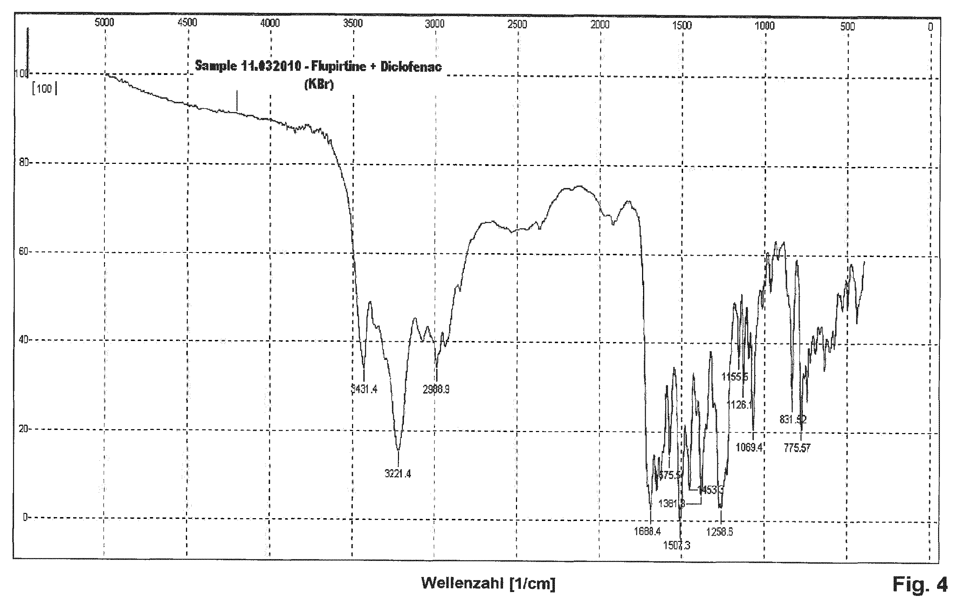

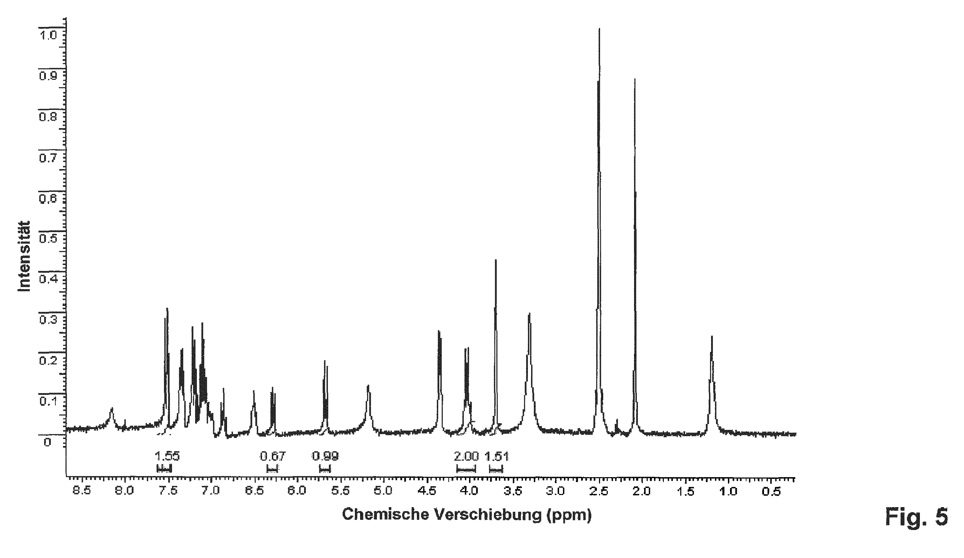

Die Struktur des Multikomponentenkristalls wurde durch IR-Spektrum (Fig. 4) und 1H-NMR-Spektrum (DMSO-d6, 400 MHz) (Fig. 5) bestätigt.

### Beispiel 2: Analyse des Multikomponentenkristalls mittels Röntgen-Pulverdiffraktometrie

Die räntgen-pulverdiffraktometrische Analyse der gemäß Beispiel 1 erhaltenen Verbindung wurde mit einem D8 Advance Pulverdiffraktometer der Firma Bruker AXS durchgeführt, dessen Spezifikationen zusammen mit den Messparametern in der Tab. 1 zusammengefasst sind:

**Tab. 1: Gerätespezifikationen und Messparameter für die Röntgen-Pulverdiffraktometrie**

| Gerätespezifikationen | |
|---|---|
| Monochromator | Johansson-Germanium-Einkristall |
| Detektor | PSD-LynxEyeDatenerfassungsbericht 4° 2θ |
| Probenträger | Kapillarprobenträger mit einem Kapillardurchmesser von 0,5 mm |

| Messparameter | |
|---|---|
| Strahlung | Cu Kα₁ |
| Generator | 40 kV, 40 mA |
| Winkelbereich | 3° bis 80° 2θ |
| Schrittweite | 0,00922° 2θ |
| Messzeit | 3 Sek./Schritt |

Das auf diese Weise ermittelte Röntgen-Pulverdiffraktogramm ist in Fig. 1 dargestellt. Ein Vergleich zwischen den Diffraktogrammen des Multikomponentenkristalls und den zu seiner Herstellung verwendeten Komponenten ([2-Amino-6-(4-fluoro-benzylamino)-pyridin-3-yl]-carbamidsäureethylester und 2-[2-[(2,6-Dichlorphenyl)-amino]-phenyl]-essigsäure ist in Fig. 2 gezeigt.

Die Signallagen des Röntgen-Pulverdiffraktogramms für den gemäß Beispiel 1 erhaltenen Multikomponentenkristall aus Flupirtin und Diclofenac sind in Tab. 2 zusammengefasst.

**Tab. 2: Signallagen des Röntgen-Pulver-Diffraktometrie-Spektrums für Flupirtin-Diclofenac aus Beispiel 1**

| **2Theta / °** | **relative Intensität / %** |
|---|---|
| 4,90 | 47 |
| 6,14 | 67 |
| 7,67 | 38 |
| 11,06 | 33 |
| 12,29 | 28 |
| 14,56 | 40 |
| 19,55 | 38 |
| 20,85 | 24 |
| 22,48 | 46 |
| 24,23 | 48 |
| 24,75 | 39 |

### Beispiel 3: Analyse des Multikomponentenkristalls mittels DSC (Differential Scanning Calorimetry; Dynamische Differenzkalorimetrie)

Das Thermogramm der gemäß Beispiel 1 erhaltenen Verbindung wurde mit einem NETZSCH DSC 204 F1 Phönix gemessen. Eine Angabe über die Spezifikationen wird in der Tab. 3 gegeben:

**Tab. 3: Gerätespezifikationen und Messparameter für die DSC (Dynamische Differenzkalorimetrie)**

| Gerätespezifikationen | |
|---|---|
| Messfühler | τ-Sensor |
| Ofen | Silberblock mit Miniatur-Mantelheizleiter |
| Kühlsystem | Mechanische Kühlung (Intracooler) |
| Probenträger | Aluminiumprobentiegel, Tiegeldurchmesser 6 mm |

| Messparameter | |
|---|---|
| Heizrate | 10°C/min |
| Temperaturprogramm | 20 °C - 300 °C |
| Schutzgas | Stickstoff |
| Gasdurchflussrate | 20 mL / min |
| Probenmasse | 7,872 mg |

Die Auswertung der Thermogramme wurde mit dem Programm Proteus (Vers. 4.8.5) der Firma NETZSCH durchgeführt. Das erhaltene Thermogramm ist in Fig. 3 gezeigt.

### Beispiel 4: Analyse des Multikomponentenkristalls mittels ¹³C CP/MAS-NMR

Alle Spektren wurden an einem Avance 400 (Firma BRUKER, Rheinstetten) bei einer ¹³C Resonanzfrequenz von 100,62 MHz gemessen. Dabei rotierten die Proben in einem 4mm-Doppelresonanz-Probenkopf mit einer Rotationsfrequenz von 10.0 kHz ("magic angle spinning", MAS). Für das CP-Experiment ("cross polarization", CP) wurde ein ¹H 90° Puls von 5.23 µs und ein Kontaktpuls von 10 ms Dauer verwendet. Die spektrale Weite betrug 250 ppm (25252 Hz).
Es wurden 13000 FIDs mit einer Wiederholzeit von 5.0 s akkumuliert. Dabei wurden 1600 Datenpunkte aufgenommen und mit einer Gesamtpunktzahl von 16384 ("zero-filling") Fourier-transformiert.
Die chemische Verschiebung bezieht sich auf Tetramethylsilan(TMS, s_{TMS}= 0.0 ppm). Als Referenz wurde nach jedem Experiment Adamantan als sekundärer, externen Standard gemessen (s_{Adamantan}= 28.72, 37.77 ppm).

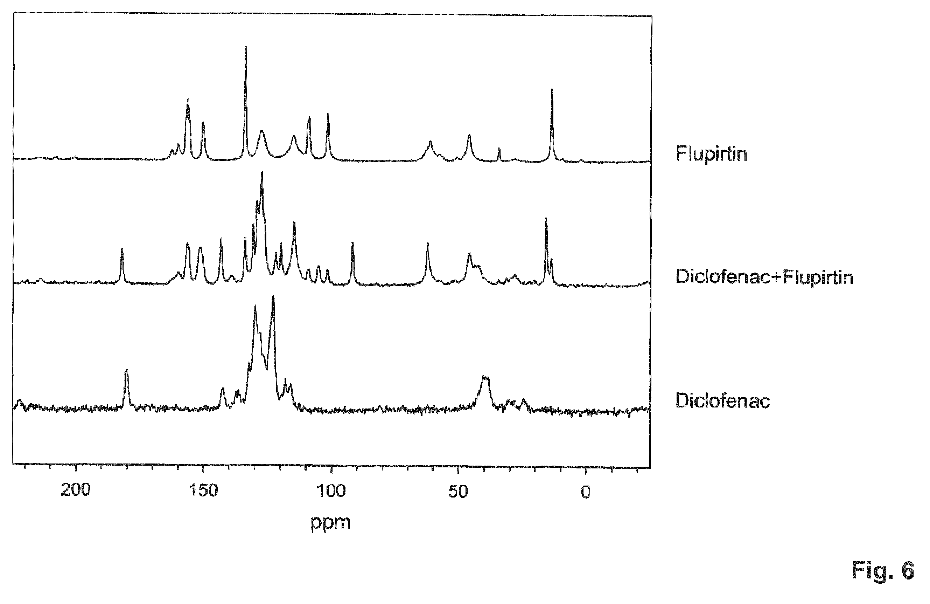

Die ¹³C-CP/MAS-NMR-Spektren des Multikomponentenkristalls sowie der Ausgangskomponenten ([2-Amino-6-(4-fluoro-benzylamino)-pyridin-3-yl]-carbamidsäureethylester und 2-[2-[(2,6-Dichlorphenyl)-amino]-phenyl]-essigsäure sind in Fig. 6 zu sehen.

### Beispiel 5: Weichkapseln enthaltend 150 mg des erfindungsgemäßen Multikomponentenkristalls aus ([2-Amino-6-(4-fluoro-benzylamino)-pyridin-3-yl-carbamidsäureethylester und 2-[2-[(2,6-Dichlorphenyl)-amino]-phenyl]-essigsäure

150 mg des erfindungsgemäßen Multikomponentenkristalls aus Flupirtin und Diclofenac, hergestellt nach Beispiel 1 werden gegebenenfalls unter leichter Erwärmung mit 300 mg Diethylenglycolmonoethylether, 100 mg Polyethylenglycol und 15 mg Polyvinylpyrrolidon und 90 mg Labrasol® gemischt und in Weichgelatinekapseln abgefüllt.

## Patentansprüche

1. Multikomponentenkristall, **dadurch gekennzeichnet, dass** er aus ([2-Amino-6-(4-fluoro-benzylamino)-pyridin-3-yl]-carbamidsäureethylester (Flupirtin) und 2-[2-[(2,6-Dichlorphenyl)-amino]-phenyl]-essigsäure (Diclofenac) besteht.

2. Multikomponentenkristall nach Anspruch 1, wobei das molare Verhältnis von ([2-Amino-6-(4-fluoro-benzylamino)-pyridin-3-yl]-carbamidsäureethylester und 2-[2-[(2,6-Dichlorphenyl)-amino]-phenyl]-essigsäure im Bereich von 0,9 : 1,0 bis 1,1 : 1,0 liegt.

3. Multikomponentenkristall nach Anspruch 1 oder 2, **gekennzeichnet durch** ein Röntgenpulverdiffraktogramm mit einem charakteristischen Peak bei 2θ = 6,1±0,2°.

4. Multikomponentenkristall nach Anspruch 3, zusätzlich **gekennzeichnet durch** ein Röntgenpulverdiffraktogramm mit einem charakteristischen Peak bei 2θ = 4,9±0,2°, 7,7±0,2°, und 19,6±0,2°.

5. Multikomponentenkristall nach Anspruch 3 oder 4, zusätzlich **gekennzeichnet durch** ein Röntgenpulverdiffraktogramm mit einem charakteristischen Peak bei 2θ = 11,1±0,2°, 12,3±0,2°, 14,6±0,2°, 20,9±0,2°, 22,5±0,2°, 24,2±0,2° und 24,8±0,2°.

6. Multikomponentenkristall nach einem der Ansprüche 1 bis 5, **gekennzeichnet durch** ein DSC-Thermogramm mit einer Schmelzendotherme im Bereich von 100 bis 115 °C mit einer Onset-Temperatur von 96,9 ± 2 °C und mit einem Peakmaximum bei 107,2 °C ± 3 °C.

7. Verfahren zur Herstellung eines Multikomponentenkristall nach einem der Ansprüche 1 bis 6 umfassend die Schritte:
a) Lösen von ([2-Amino-6-(4-fluoro-benzylamino)-pyridin-3-yl]-carbamidsäureethylester und 2-[2-[(2,8-Dichlorphenyl)-amino]-phenyl]-essigsäure in einem molaren Verhältnis von 1,0: 0,9 bis 1,0: 1,1 in einem inerten organischen Lösungsmittel und
b) Auskristallisieren der Komplexverbindung.

8. Pharmazeutische Zubereitung umfassend einen Multikomponentenkristall nach einem der Ansprüche 1 bis 6 als Wirkstoffkombination.

9. Pharmazeutische Zubereitung nach Anspruch 8 in Form einer Weichkapsel.

10. Transdermale pharmazeutische Zubereitung umfassend einen Multikomponentenkristall nach einem der Ansprüche 1 bis 6 als Wirkstoff.

11. Orale pharmazeutische Zubereitung nach Anspruch 8 oder 9 oder transdermale pharmazeutische Zubereitung nach Anspruch 10, wobei die pharmazeutische Zubereitung 50 bis 1000 mg eines Multikomponentenkristalls gemäß einem der Ansprüche 1 bis 6 je Verabreichungseinheit als Wirkstoff enthält.

12. Verwendung eines Multikomponentenkristalls nach einem der Ansprüche 1 bis 6, einer oralen pharmazeutischen Zubereitung nach Anspruch 8 oder 9 und/oder einer transdermalen pharmazeutischen Zubereitung nach Anspruch 10 zur Behandlung und Vorbeugung von akuten und chronischen Schmerzen, einschließlich neuropathischen Schmerzen, Nervenschmerzen, durch Krebserkrankungen verursachte Schmerzen, Spannungskopfschmerz, vasomotorische und Migräne-Kopfschmerzen, Schmerzzuständen nach Operationen, nach Verletzungen, Verbrennungen, Verätzungen, bei Dysmenorrhoe, Zahnschmerzen und arthritischen Schmerzen sowie für die Behandlung von Entzündungsschmerz oder Schmerzen unklarer Genese.

13. Verwendung eines Multikomponentenkristalls nach einem der Ansprüche 1 bis 6, einer oralen pharmazeutischen Zubereitung nach Anspruch 8 oder 9 und/oder einer transdermalen pharmazeutischen Zubereitung nach Anspruch 10 zur Behandlung und Vorbeugung von Muskelverspannung, Muskelspasmus und Muskelsteifheit, insbesondere zur Behandlung von Rückenschmerzen unklarer Genese.

14. Verwendung eines Multikomponentenkristalls nach einem der Ansprüche 1 bis 6, einer oralen pharmazeutischen Zubereitung nach Anspruch 8 oder 9 und/oder einer transdermalen pharmazeutischen Zubereitung nach Anspruch 10 zur gleichzeitigen Behandlung von Schmerzen verschiedener Ursachen, insbesondere entzündlichen Schmerzen, und Schmerzen bedingt durch Muskelverspannungen

15. Verfahren zur Herstellung einer pharmazeutischen Zubereitung umfassend das Mischen einer Komplexverbindung nach einem der Ansprüche 1 bis 6 mit einem Lösungsmittel auf Glykolbasis, einem Lösungsvermittler und einem Viskosität verleihenden Mittel wie PVP und Einbringen der Mischung in eine Weichgelatinekapsel.
